# EUROPÄISCHE PATENTSCHRIFT

(11) **EP 2 247 648 B1**
(45) Veröffentlichungstag und Bekanntmachung des Hinweises auf die Patenterteilung: **03.01.2018**
(21) Anmeldenummer: 09707491.8
(22) Anmeldetag: 05.02.2009
(51) Int. Cl.: C08G 69/20, C07D 201/14

(54) **VERFAHREN ZUR HERSTELLUNG VON LACTAMATEN DURCH DÜNNSCHICHTVERDAMPFUNG**
METHOD FOR PRODUCING LACTAMATES BY WAY OF THIN FILM EVAPORATION
PROCÉDÉ DE PRODUCTION DE LACTAMATES PAR ÉVAPORATION EN COUCHE MINCE

(30) Priorität: 08.02.2008 DE 102008000259
(43) Veröffentlichungstag der Anmeldung: 10.11.2010
(73) Patentinhaber: LANXESS Deutschland GmbH, 50569 Köln (DE)
(72) Erfinder: LAUFER, Wilhelm, 67158 Ellerstadt (DE); KASPER, Hans, 68782 Brühl (DE); SCHATTNER, Stefan, 67593 Bermersheim (DE); ALLGOEWER, Klaus, 67071 Ludwigshafen (DE); KRAY, Bernd, 67346 Speyer (DE); WUEHR, Michael, 69493 Hirschberg (DE)
(86) Internationale Anmeldenummer: PCT/EP2009/051323
(87) Internationale Veröffentlichungsnummer: WO 2009/098259

(56) Entgegenhaltungen:
- EP-A1- 0 238 143
- DE-A1- 1 495 132
- DE-B- 1 204 821
- US-A- 3 681 293

## Beschreibung

Die vorliegende Erfindung betrifft ein Verfahren zur Herstellung von Lactamaten durch Umsetzung von Alkoholaten mit Lactamen.

Werden Lactame in Gegenwart alkalischer Substanzen erwärmt, so polymerisieren sie zu den entsprechenden Polyamiden. Geeignete und häufig verwendete Katalysatoren hierfür sind Lactamate, welche in Kombination mit Aktivatoren auf Isocyanatbasis verwendet werden.

Verfahren zur Herstellung von lactamathaltigen Katalysatorsystemen sind an sich bekannt.

So beschreibt beispielsweise die US 4,115,399 die Herstellung einer Lösung, welche zur Katalyse der anionischen Polymerisation von 2-Pyrrolidinon geeignet ist. Die Katalysatorlösung wird durch batchweise Umsetzung eines Alkalimetallhydroxids mit einem Überschuss 2-Pyrrolidinon und anschließender Überführung der resultierenden Mischung zur Dehydratisierung bei erhöhter Temperatur unter gleichzeitigem Strippen hergestellt. Die Umsetzung wird dabei in einer Reaktionszone, beispielsweise einem Rührreaktor, durchgeführt. Nach der Umsetzung wird das erhaltene Reaktionsgemisch in eine Destillationsvorrichtung überführt, in der das durch die Umsetzung des Alkalimetallhydroxids mit dem Überschuss 2-Pyrrolidinon gebildete Wasser aus der Reaktionsmischung entfernt wird. Das Verfahren gemäß US 4,115,399 umfasst somit zumindest zwei unterschiedliche Reaktionsstufen. Die Umsetzung des 2-Pyrrolidons und des Alkalimetallhydroxids erfolgt bei einer Temperatur zwischen 25 und 100 °C, während die destillative Aufarbeitung des Umsetzungsprodukt aus 2-Pyrrolidinon und dem Alkalimetallhydroxid bei 75 bis 150 °C erfolgt.

EP 0 438 762 A1 beschreibt eine Katalysatorlösung für die anionische Polymerisation von Lactamen zu Polyamid, die neben spezifischen Modifizierungsmitteln das zu polymerisierende Lactam, ein Alkalilactamat und 2-Pyrrolidinon enthält. Die zur Polymerisation einzusetzende Lösung wird durch Mischen des Lactams und des 2-Pyrrolidinons mit den einzelnen Modifizierungsmitteln und anschließender Zugabe eines festen Alkalialkoholats in einem ersten Verfahrensschritt hergestellt. Diese Mischung wird dann anschließend bei erhöhter Temperatur und unter vermindertem Druck einer Destillation unterzogen, bei welcher der bei der Reaktion zwischen Lactam und Alkalialkoholat freiwerdende Alkohol abdestilliert wird.

DE-OS 2 035 733 beschreibt die Herstellung von Polyamiden durch anionische Polymerisation von Lactamen, wobei die Polymerisation in Gegenwart von katalytisch wirkenden Lösungen von (Erd-)-Alkalilactamaten in α-Pyrrolidinon durchgeführt wird. Die diskontinuierliche Herstellung der katalytisch-wirkenden Lösung erfolgt durch Umsetzung eines Lactams mit einer alkoholischen (Erd-)Alkalialkoholat-Lösung unter Erwärmung, anschließender Zugabe von α-Pyrrolidinon und destillativer Entfernung des durch die Reaktion des Lactams mit dem Alkoholat gebildeten Alkohols.

US 3,575,938 offenbart ein Verfahren zur Hestellung einer Katalysatorlösung für die Herstellung von Polyamiden durch anionische Polymerisation. Die Katalysatorlösung wird durch ein zweistufiges Verfahren der Umsetzung von einem Metallhydrid, Metallhydroxid, Metallalkylat, Metallalkoxid, Metallamid oder Metallcarbonat mit einem Lactammonomer bei vorzugsweise erhöhter Temperatur und einer anschließenden Destillation als zweitem Verfahrensschritt hergestellt.

Aus DE 1204821, EP 0238143 und DE 1495132 sind Verfahren zur Herstellung von Lactamaten bekannt, bei denen Lactam und Alkohole beim Temperaturen > 80 °C eingesetzt werden, wodurch es zur unerwünschten Bildung von Oligomeren/Polymeren kommt.

Nachteilig an diesen Verfahren sind zum einen die hohen Investitionskosten, die sich durch die spezifische Verfahrensvorrichtung aus einem Reaktor für die Umsetzung des Lactams mit der Base und einer Destillationsvorrichtung ergeben.

Darüber hinaus sind die resultierenden Produktbeschaffenheiten des Katalysators nicht immer zufrieden stellend. So kann es bei den beschriebenen Herstellungsverfahren zu einer Trübung des Katalysatorsystems aufgrund einer Polymer- und Oligomerbildung kommen, wodurch erhöhte Reinigungskosten aufgrund der Entfernung der entstandenen Oligomerisate bzw. Polymerisate aus den verwendeten Vorrichtungen resultieren.

Darüber hinaus können in den resultierenden Katalysatorsystemen hohe Alkoholgehalte durch die Verwendung von Alkoholaten als Base auftreten, was sich auf die Katalysatoreignung negativ auswirkt und in den resultierenden Polyamidprodukten zu einer Lunkerbildung führt.

Ein weiterer Nachteil der insbesondere diskontinuierlich arbeitenden Verfahren ist, dass nur geringe Durchsätze erreicht werden. Bei den kontinuierlich arbeitenden Verfahren ist darüber hinaus nachteilig, dass im Allgemeinen hohe Vorlaufmengen bei der Destillation zur Feedeinstellung erforderlich sind.

Demgemäß liegt der vorliegenden Erfindung die Aufgabe zugrunde, ein Verfahren zur Herstellung von Katalysatoren zur Polymerisation von Lactamen zu Polyamiden bereitzustellen, welches im Allgemeinen zu einer Vermeidung der oben genannten Probleme führt.

Insbesondere soll das Verfahren vorzugsweise eine kostengünstige Herstellung unter gleichzeitigem Erhalt eines qualitativ hochwertigen Katalysators ermöglichen. Dabei soll das Verfahren insbesondere zu einem Katalysatorsystem führen, der einen geringen Anteil an Oligomer bzw. Polymer aufweist, so dass die üblichen Reinigungsschritte nicht mehr erforderlich sind. Ferner soll der resultierende Katalysator vorzugsweise einen geringen Anteil an Alkohol aufweisen, da größere Mengen an Alkohol zu einer verringerten Stabilität des Katalysators führen und die Anwesenheit von Alkohol bei der anionischen Polymerisation zu Polyamid zu einer Gasbildung führt, die wiederum eine Bildung von Lunkern im Endprodukt auslösen kann. Dabei sollte das Verfahren vorzugsweise hohe Durchsätze ermöglichen und vorzugsweise ein schnelles Erreichen der Betriebsbedingungen gewährleisten, so dass niedrige Feedvorlaufmengen erforderlich sind.

Die Lösung dieser Aufgabe geht aus von einem Verfahren zur Herstellung von Lactamaten durch Umsetzung von mindestens einem Alkoholat mit mindestens einem Lactam.

Das erfindungsgemäße Verfahren ist dann dadurch gekennzeichnet, dass eine Reaktionsmischung, enthaltend mindestens ein Alkoholat und mindestens ein Lactam, einer Reaktivdestillation auf einem Dünnschichtverdampfer unterworfen wird, wobei die folgenden Verfahrensschritte durchlaufen werden:
(a) Bereitstellen mindestens einer alkoholischen Alkoholat-Lösung;
(b) Bereitstellen mindestens einen Lactams;
(c) Einbringen des mindestens einen Lactams in die alkoholische Alkoholat-Lösung unter Erhalt einer Eduktmischung;
(d) Homogenisierung der in Verfahrensschritt (c) erhaltenen Eduktmischung bei einer Temperatur von 30 - 70 °C, die so gewählt ist, dass es im Wesentlichen zu keiner Reaktion des Lactams mit dem Alkoholat kommt, unter Erhalt einer homogenisierten Eduktmischung;
(e) Zuspeisung der homogenisierten Eduktmischung in einen Dünnschichtverdampfer, vorzugsweise über den Kopf des Dünnschichtverdampers;
(f) Reaktivdestillation der zugespeisten Eduktmischung, gegebenenfalls unter gleichzeitigem Strippen der Eduktmischung im Gegenstrom; und
(g) Erhalt des Lactamats am Boden des Dünnschichtverdampfers

Unter einer Reaktivdestillation wird im Rahmen der vorliegenden Erfindung die Integration einer Reaktion (Bildung des Katalysators Lactamat) und eines Trennprozesses (destillative Abtrennung des durch die Reaktion gebildeten Alkohols) verstanden.

Die in dem erfindungsgemäßen Verfahren verwendete Reaktionsmischung enthält mindestens ein Lactam. Hierzu geeignete Lactame sind Verbindungen der allgemeinen Formel 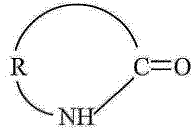 wobei R einer Alkylengruppe mit vorzugsweise 3 bis 13 Kohlenstoffatomen, besonders bevorzug 5 bis 7 Kohlenstoffatomen, entspricht. Beispiele geeigneter Lactame sind Caprolactame, insbesondere ε-Caprolactam, Butyrolactam, Önanthlactam, Capryllactam, Lauryllactam, α-Pyrrolidinon, Piperidon, Valerolactam sowie deren Mischungen. Im Rahmen der vorliegenden Erfindung ist ε-Caprolactam besonders bevorzugt. Das verwendete Lactam wird vorzugsweise in Schmelzform verwendet, es kann jedoch auch in Schuppenform verwendet werden .

Insbesondere ist es bevorzugt, dass in dem erfindungsgemäßen Verfahren nur ein einziges Lactam verwendet wird, d. h., dass in dem erfindungsgemäßen Verfahren keine Mischung von zwei oder mehreren Lactamen Anwendung findet.

Da das im erfindungsgemäßen Verfahren verwendete Lactam selten quantitativ mit dem Alkoholat unter Ausbildung des Lactamats reagiert, liegt in dem durch das erfindungsgemäße Verfahren erhältlichen Produkt häufig zusätzlich unumgesetztes Lactam vor. Dieses kann in dem durch das erfindungsgemäße Verfahren erhältlichen Produkt je nach Wahl des Lactams eine Lösemittelfunktion übernehmen. Im Allgemeinen wirkt das unumgesetzte Lactam in der Polyamidsynthese nicht störend, führt jedoch gegebenenfalls zu Nebenreaktionen, insbesondere zu einer Copolyamidbildung. Demgemäß kann es im Rahmen der vorliegenden Erfindung bevorzugt sein, wenn das in der Reaktionsmischung zu verwendende Lactam auf das später zu polymerisierende Lactam abgestimmt wird, so dass eine spätere Copolyamidbildung vermieden werden kann. In diesem Fall ist die Verwendung eines einzigen Lactams in dem erfindungsgemäßen Verfahren bevorzugt.

Die für die Lactamatbildung eingesetzten Alkoholate weisen bevorzugt eine höhere Basizität auf, als das Lactamatanion, dessen Bildung zu bewirken ist. Beispiele für geeignete Alkoholate sind Alkali- oder Erdalkalimetallalkoholate von niedrigsiedenden Alkoholen, vorzugsweise Alkali- oder Erdalkalimetallmethylate oder -ethylate, besonders bevorzugt Natriummethylat und -ethylat. Beispiele weiterer geeigneter Basen sind die Alkoholate von Lithium, Kalium, Magnesium, Calcium, Strontium, Barium oder auch Tetraalkylammoniumalkohotate.

Die vorgesehenen Alkoholate können in dem erfindungsgemäßen Verfahren in reiner Form als Alkoholatpulver oder als Lösung oder Suspension eingesetzt werden. Als Lösungsmittel zur Herstellung von Lösungen oder Suspensionen sind im Prinzip alle niedrigsiedende Lösungsmittel geeignet, die die Umsetzung nicht stören. Bevorzugt werden niedrigsiedende Alkohole als Lösungsmittel für die Alkoholate verwendet. Der Gehalt an Alkoholat in den bevorzugt alkoholischen Lösungen beträgt weniger als 100 Gew.-%, besonders bevorzugt 20 bis 40 Gew.-%.

In dem erfindungsgemäßen Verfahren beträgt das Gewichts-Verhältnis von Lactam zu Alkoholat vorzugsweise etwa 1 : 1 bis 40 : 1, besonders bevorzugt etwa 16 : 1 bis 32 : 1, insbesondere etwa 25 : 2. Das gewählte Verhältnis hängt im Wesentlichen davon ab, wie der Schmelzpunkt des entstehenden Produktes ist. Solange der Schmelzpunkt derart ist, dass noch auf einem Dünnschichtverdampfer gearbeitet werden kann, kann insbesondere auch mit einem stöchiometrischen Gewichts-Verhältnisses zwischen Lactam und Alkoholat gearbeitet werden.

Die erfindungsgemäß vorgesehene Reaktivdestillation wird auf einem Dünnschichtverdampfer durchgeführt. Unter einem Dünnschichtverdampfer wird im Rahmen der vorliegenden Erfindung eine Vorrichtung zur destillativen Trennung von Stoffgemischen verstanden, wobei das zu trennende Stoffgemisch durch Abrieseln, Einwirken von Zentrifugalkraft oder besonders konstruierte Wischer zu dünnen Schichten auf heißen Flächen verteilt wird. Wenn die zu trennende Flüssigkeit durch Abrieseln zu dünnen Schichten verteilt wird, so wird der verwendete Dünnschichtverdampfer häufig auch als Fallfilmverdampfer oder Rieselkolonne bezeichnet, welche im Rahmen der vorliegenden Erfindung verwendet werden können.

In der vorliegenden Erfindung findet auf dem Dünnschichtverdampfer eine Reaktion des mindestens einen Alkoholats mit dem mindestens einem Lactam unter Ausbildung des katalytisch-wirkenden Lactamats statt. Bei dieser Reaktion wird der korrespondierende Alkohol freigesetzt, der sodann unmittelbar auf dem Dünnschichtverdampfer der Reaktionsmischung entzogen wird. Insbesondere ist im Rahmen der vorliegenden Erfindung eine Anwendung einer Reaktionsstufe vor der Reaktivdestillation nicht vorgesehen, in welcher es aufgrund der dort gewählten Temperatur zu einer Umsetzung der Base mit Lactam zum Lactamat kommt.

Der im Rahmen des vorliegenden Verfahrens verwendete Dünnschichtverdampfer ist vorzugsweise mit einem Rührwerk ausgestattet.

Die Verfahrensbedingungen des Dünnschichtverdampfers, insbesondere die Wahl von Temperatur und Druck, hängen von dem verwendeten Lactam und dem verwendeten Alkoholat ab und sind entsprechend anzupassen.

Bei der Verwendung von ε-Caprolactam und Natriummethylat bzw. einer methanolischen oder allgemein einer alkoholischen Natriummethylat-Lösung wird die Reaktivdestillation vorzugsweise bei mindestens einer der folgenden Temperatur- und/oder Druckbedingungen durchgeführt:
Die genauen Betriebsbedingungen der Reaktivdestillation hängen naturgemäß vom eingesetzten Lactamat, insbesondere dessen Schmelzpunkt ab. Die Reaktivdestillation wird vorzugsweise bei einer Betriebstemperatur des Mantels des Dünnschichtverdampfers von vorzugsweise 80 bis 180 °C, bevorzugt von 80 bis 130 °C, durchgeführt. Der Kondensator des Dünnschichtverdampfers wird bei einer Temperatur von vorzugsweise -10 bis 30 °C, insbesondere 5 bis 15 °C, betrieben. Die Produkttemperatur in der Vorlage beträgt vorzugsweise 30 bis 70 °C, bevorzugt 40 bis 60 °C, insbesondere 45 bis 55 °C. Durch diese Temperatureinstellung in der Vorlage ist es gewährleistet, dass es zu im Wesentlichen keiner Umsetzung des Lactams mit der Base kommt. Die Temperatur der Reaktionsleitung in der Überleitung zum Dünnschichtverdampfer kann bereits in Richtung der Reaktionstemperatur der Reaktivdestillation angepasst werden. Darüber hinaus wird die Reaktivdestillation vorzugsweise so durchgeführt, dass die Produkttemperatur am Auslauf 75 bis 120 °C, besonders bevorzugt 80 bis 90 °C, insbesondere 75 bis 85 °C, beträgt. Ferner wird die Reaktivdestillation bei einer Kontakttemperatur von vorzugsweise 80 bis 150 °C, besonders bevorzugt 90 bis 100 °C, durchgeführt. Die Reaktivdestillation wird vorzugsweise bei einem Druck von 50 bis 900 mbar, besonders bevorzugt von 100 bis 300 mbar durchgeführt.

Die auf dem Reaktivverdampfer angewendeten Kontaktzeiten der Eduktmischung betragen im Allgemeinen von 2 bis 60 sec, besonders bevorzugt von 5 bis 20 sec, besonders bevorzugt von 10 bis 20 sec.

Die Reaktionsmischung aus Alkoholat bzw. alkoholischer Alkoholatlösung und Lactam wird dem Dünnschichtverdampfer vorzugsweise am Kopf zugeführt.

Zur Entfernung des während der Reaktion zwischen Lactam und Alkoholat gebildeten Alkohols und gegebenenfalls des Lösemittelalkohols hat es sich als vorteilhaft erwiesen, wenn in dem Dünnschichtverdampfer das Reaktionsgemisch zusätzlich einem Strippvorgang unterworfen wird. In einer weiteren bevorzugten Ausführungsform des erfindungsgemäßen Verfahrens wird die Reaktionsmischung daher während der Reaktivdestillation mit einem inerten Gas gestrippt. Hierzu geeignete inerte Gase sind vorzugsweise ausgewählt aus der Gruppe, bestehend aus Stickstoff, Argon und Helium. Wenn ein Strippvorgang vorgesehen ist, so erfolgt dieser vorzugsweise im Gegenstrom zur Flussrichtung der Reaktionsmischung. In einer Ausführungsform wird das Strippgas dabei am Boden dem Dünnschichtverdampfer zugeführt und fließt entgegen dem Strom der Reaktionsmischung, die vorzugsweise am Kopf dem Dünnschichtverdampfer zugeführt wird.

In dem erfindungsgemäßen Verfahren wird das Produkt der Reaktivdestillation vorzugsweise am Boden des Dünnschichtverdampfers erhalten.

Wenn in dem erfindungsgemäßen Verfahren ein Strippen während der Reaktivdestillation vorgesehen ist, so erfolgt das Strippen mit einer Menge von vorzugsweise 0 bis 200 m³/h Strippgas, besonders bevorzugt 10 bis 50 m³/h Strippgas.

In einer weiteren bevorzugten Ausführungsform wird das Strippgas am Boden des Dünnschichtverdampfers in unmittelbarer Nähe der Lactamatentnahme eingespeist.

Für eine Verwendung des Produkts der Reaktivdestillation als Katalysator zur anionischen Polymerisation von Lactamen zu Polyamiden hat es sich als vorteilhaft herausgestellt, wenn die Reaktivdestillation so durchgeführt wird, dass der Restgehalt an Alkohol in dem resultierenden lactamathaltigen Produkt vorzugsweise höchstens 1 Gew.-%, besonders bevorzugt höchstens 0,5 Gew.-%. insbesondere höchstens 0,3 Gew.-%, beträgt.

Die Erfinder der vorliegenden Erfindung haben herausgefunden, dass das durch das erfindungsgemäße Verfahren erhältliche Produkt im Trübungstest keine Trübung in einer 10 %igen methanolischen Lösung zeigt, was mit einem allenfalls sehr geringen Restanteil an Oligo- und/oder Polymerisat korreliert.

Darüber hinaus ist es für das lactamathaltige Produkt vorteilhaft, wenn dessen Verweilzeiten während der Reaktivdestillation in dem Dünnschichtverdampfer möglichst gering sind. Auch dieses wird durch das erfindungsgemäße Verfahren geleistet.

Aus ökonomischen Gründen ist es dabei bevorzugt, wenn der Durchsatz des Dünnschichtverdampfers etwa 10 bis 100 kg/h · m², besonders bevorzugt etwa 50 kg/h · m², beträgt.

In einer besonders bevorzugten Ausführungsform umfasst das erfindungsgemäße Verfahren die folgenden Verfahrensschritte:
Wenn in dem erfindungsgemäßen Verfahren ε-Caprolactam und Natriummethylat verwendet werden, so findet die in Verfahrensschritt (d) vorgesehene Homogenisierung vorzugsweise bei einer Temperatur von 44 bis 50 °C statt. Bei dieser Temperatur findet im Wesentlichen noch keine Umsetzung statt.

Vorrichtungen zur Durchführungen der Homogenisierung sind dem Fachmann an sich bekannt. Geeignet sind beispielsweise übliche Rührreaktoren.

Die Zuspeisung der in Verfahrensschritt (d) erhaltenen Reaktionsmischung auf den Dünnschichtverdampfer erfolgt vorzugsweise sobald die Reaktionsmischung homogenisiert ist. Dieses ist beispielsweise daran zu erkennen, dass eine klare Lösung erhalten wird.

Während der Homogenisierung in Verfahrensschritt (d) erfolgt vorzugsweise im Wesentlichen keine Reaktion zwischen dem Lactam und dem Alkoholat.

An den Verfahrensschritt (g) kann sich ferner eine Konfektionierung des erhaltenen Lactamats anschließen. Diese erfolgt vorzugsweise auf einer Schuppenwalze oder einem Pastillierband, wobei das resultierende Produkt in Form von Schuppen oder Pellets erhalten wird.

Da das Produkt des erfindungsgemäßen Verfahrens sauerstoffempfindlich ist, ist es bevorzugt, wenn in dem erfindungsgemäßen Verfahren zumindest einzelne Verfahrensschritte unter inerten Bedingungen durchgeführt werden. In einer weiteren Ausführungsform des erfindungsgemäßen Verfahrens werden jedoch alle Verfahrensschritte, gegebenenfalls einschließlich der Konfektionierung, unter inerten Bedingungen durchgeführt und das erhaltene, gegebenenfalls konfektionierte Produkt, unter inerten Bedingungen aufbewahrt. Die Durchführung der einzelnen Verfahrensschritte unter inerten Bedingungen ist dem Fachmann an sich bekannt. Üblicherweise werden hierzu die verwendeten Vorrichtungen und Apparaturen evakuiert und anschließend mit einem Inertgas, beispielsweise Stickstoff oder Argon, wieder bis zum Druckausgleich beaufschlagt. Je nach Empfindlichkeit des resultierenden Lactamats kann dabei dieser Vorgang ein- oder mehrfach wiederholt werden.

Das erfindungsgemäße Verfahren kann sowohl batchweise als auch kontinuierlich durchgeführt werden. Die kontinuierliche Fahrweise ist jedoch bevorzugt.

Dem erhaltenen Lactamat können auch eigenschafts- und anwendungsbedingte Zusatzstoffe, welche die anschließende Lactampolymerisation nicht oder nur unwesentlich beeinträchtigen, zugefügt werden. Hierbei handelt es sich beispielsweise um Entformungsmittel, Entschäumer, Hitze-, Licht- und/oder Oxidations-Stabilisatoren, Nukleierungsmittel, Tracer, optische Aufheller, Weichmacher, Schlagzähmittel, Füll- und Verstärkerstoffe, Öle, gegebenenfalls aminoterminierte bzw. einbaufähige Polyether und Farbmittel (Pigmente).

Durch das erhaltene lactamathaltige Produkt kann sowohl in kontinuierlichen als auch in diskontinuierlichen Verfahren die Polymerisation von Lactamen zu Polyamiden direkt ausgelöst werden. Die Verwendung des erhaltenen lactamathaltigen Produkts kann insbesondere in Extrusions-, Spritzguss-, Pultrusions-, Monomerguss-, Resintransfermolding-, Reactioninjectionmolding- und Rotormolding-Verfahren sowie zur Herstellung von Verbundwerkstoffen mit Polyamid als Matrix verwendet werden.

Das erfindungsgemäße Verfahren ermöglicht eine effiziente Herstellung von lactamathaltigen Katalysatorsystemen, wobei hohe Vorlaufmengen zur Einstellung des Feeds bei der Destillation nicht erforderlich sind. Die Bildung von Polymerisaten und Oligomeren wird minimiert und ermöglicht somit niedrige Reinigungskosten verwendeter Vorrichtungen und Apparaturen. Das resultierende Produkt weist darüber hinaus niedrige Restalkoholkonzentrationen auf.

Die vorliegende Erfindung wird anhand des folgenden Ausführungsbeispiels näher beschrieben, welches jedoch keine Beschränkung der Erfindung darstellt.

### Erfindungsgemäßes Ausführungsbeispiel:

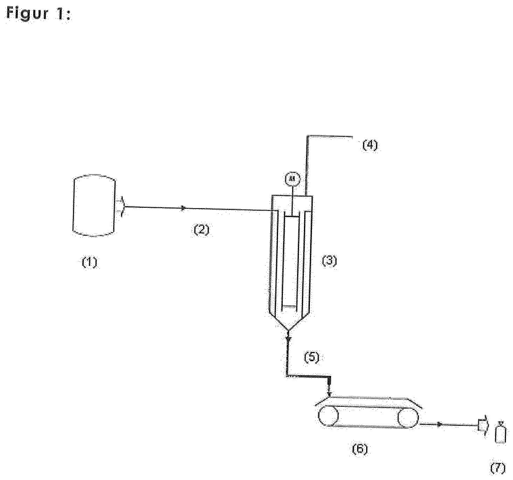

Figur 1 zeigt ein Verfahrensfließbild für das erfindungsgemäße Verfahren, welches in dem nachfolgenden Verfahren bzw. Anwendung findet.

In einem Behälter (1) wird eine homogenisierte Eduktmischung aus 797 kg ε-Caprolactam und 203 kg 30%iger Natriummethylat-Lösung in Methanol bei einer Temperatur von 47 °C hergestellt. In dieser homogenisierten Eduktmischung findet zunächst im Wesentlichen keine Reaktion des Alkoholats mit dem Lactam statt. Über die Leitung (2) wird das homogenisierte Reaktionsgemisch in einen Dünnschichtverdampfer (3) überführt, welcher bei den folgenden Verfahrensparametern betrieben wird:

| | |
|---|---|
| Manteltemperatur | ca. 120 °C |
| Druck | 200 mbar |
| Kondensatortemperatur | 10 °C |
| Kontakttemperatur | ca. 110 °C |
| Produkttemperatur am Auslauf | ca. 80 °C |
| Produkttemperatur in Vorlage | ca. 50 °C |
| Stickstoff | ca. 15 bis 25 m³/h (Gegenstrom) |
| Durchsatz | ca. 50 kg/h · m² |

Unter diesen Bedingungen findet die Umsetzung des Lactams mit dem Alkoholat statt.

Am Kopf des Dünnschichtverdampfers wird das Methanol als Kondensat über die Rohrleitung (4) entnommen. Das gebildete lactamathaltige wird am Boden des Dünnschichtverdampfers über eine Rohrleitung (5) entnommen und einem Pastilierband oder einer Schuppenwalze (6) zugefügt. Das Produkt (7) wird mit folgenden Spezifikationen erhalten:
Gehalt an Natriumcaprolactamat (bestimmt durch acidimetrische Titration): 17,5 bis 19,5 %.

Gehalt an Methanol (bestimmt durch Gaschromatographie): < 0,3 Ges.-%.

Bei einem Löslichkeitstest von 2 % in Caprolactam wird bei 120 °C eine klare Lösung erhalten. Ein Löslichkeitstest bei Raumtemperatur liefert in 10 % Methanol ebenfalls eine klare Lösung.

Alle Verfahrensschritte werden unter inerter Atmosphäre durchgeführt.

### Vergleichsbeisipiel:

76 Gew.-Teile Caprolactam und 24 Gew.-Teile Natriummethylat (als 30%ige methanolische Lösung) werden bei 80 °C bzw. 110 °C (2 Versuche) gerührt. Danach wird Vakuum (bis 10 mbar) angelegt und das überschüssige Methanol abdestilliert. Die Destillationsdauer beträgt ca. 2 Stunden.

Der Methanolgehalt in dem Produkt liegt oberhalb von 0,8 %.

Bei einem Löslichkeitstest von 2 % in Caprolactam wird bei 120 °C eine trübe Lösung erhalten. Ein Löslichkeitstest bei Raumtemperatur in Methanol wurde nicht mehr durchgeführt, da eine trübe Lösung bereits bei Verwendung von Caprolactam als Lösemittel erhalten wird.

Die Vorteile des erfindungsgemäßen Verfahrens lassen sich wie folgt zusammenfassen:
1. Im Vergleich zu einem Batch-Verfahren in einem Kessel bilden sich praktisch keine Oligomere oder Polymere des Lactams, was durch ein Vergleich der oben beschriebenen Versuche gezeigt wird. Dies wird wahrscheinlich dadurch ermöglicht, dass die Umsetzung bei höherer Temperatur mit kürzerer Verweilzeit durchgeführt werden kann. Erfindungsgemäß kann der Anteil des Methanols im erhaltenen Produkt deutlich unter 1 Gew.-%, bevorzugt unter 0,5 Gew.-%, verringert werden.
   Die Anwesenheit von Methanol bei der Polymerisation zu Polyamid führt zur Gasbildung, es entstehen Lunker im Polyamid-Endprodukt, die möglichst zu vermeiden sind.
2. Weiterhin zeigt sich, dass in dem nach dem Batch-Verfahren hergestellten Material größere Mengen Oligomere vorliegen, da sich das Material beim Schmelzen in Caprolactam trübt und außerdem keine vollständige Löslichkeit in Alkoholen insbesondere in Methanol bzw. Ethanol gegeben ist. Beim Batch-Verfahren im Kessel braucht man generell längere Verweilzeiten aufgrund der Destillation, um das Methanol vollständig zu entfernen. Dadurch findet jedoch bereits teilweise eine Polymerisation des Caprolactams statt.

Die Nachteile des Vorhandenseins von Oligomeren oder Polymeren im Katalysator bestehen einerseits darin, dass höhere Kosten zur Reinigung der Vorrichtungen auftreten. Andererseits treten durch den höhern Oligomer- und Polymergehalt bei der Polymerisation Inhomogenitäten auf. Da innerhalb der Polymerisationszone unterschiedliche Polymerisationsgeschwindigkeiten auftreten, kommt es zur Bildung von Inhomogenitäten im resultierenden Polymer.

Es ist mit dem erfindungsgemäßen Verfahren überraschend gelungen, den Anteil der in der Polymerisation störenden Oligomere des Caprolactams praktisch auf einen Anteil zu reduzieren, der unterhalb der Nachweisgrenze liegt.

## Patentansprüche

1. Verfahren zur Herstellung von Lactamaten durch Umsetzung von mindestens einem Alkoholat mit mindestens einem Lactam, **dadurch gekennzeichnet, dass** eine Reaktionsmischung, enthaltend mindestens ein Alkoholat und mindestens ein Lactam, einer Reaktivdestillation auf einem Dünnschichtverdampfer unterworfen wird, wobei die folgenden Verfahrensschritte durchlaufen werden:
(a) Bereitstellen mindestens einer alkoholischen Alkoholat-Lösung;
(b) Bereitstellen mindestens eines Lactams;
(c) Einbringen des mindestens eines Lactams in die alkoholische Alkoholat-Lösung unter Erhalt einer Eduktmischung;
(d) Homogenisierung der in Verfahrensschritt (c) erhaltenen Reaktionsmischung bei einer Temperatur von 30 - 70°C, die so gewählt ist, dass es im Wesentlichen zu keiner Reaktion des Lactams mit dem Alkoholat kommt, unter Erhalt einer homogenisierten Eduktmischung;
(e) Zuspeisung der homogenisierten Eduktmischung in einen Dünnschichtverdampfer, vorzugsweise über den Kopf des Dünnschichtverdampfers;
(f) Reaktivdestillation der zugespeisten Eduktmischung, gegebenenfalls unter gleichzeitigem Strippen der Eduktmischung im Gegenstrom; und
(g) Erhalt des Lactamats am Boden des Dünnschichtverdampfers.

2. Verfahren nach Anspruch 1, **dadurch gekennzeichnet, dass** das Lactam der allgemeinen Formel 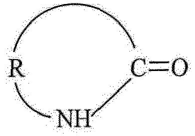 entspricht, wobei R eine Alkylengruppe mit 3 bis 13 Kohlenstoffatomen darstellt.

3. Verfahren nach Anspruch 1 oder 2, **dadurch gekennzeichnet, dass** als Alkoholat ein Alkalimetall-, Erdalkalimetall- oder Tetraalkylammoniumalkoholat verwendet wird.

4. Verfahren nach einem der Ansprüche 1 bis 3, **dadurch gekennzeichnet, dass** das Gewichts-Verhältnis von Lactam zu Alkoholat in der Reaktionsmischung 1 : 1 bis 40 : 1 beträgt.

5. Verfahren nach einem der Ansprüche 1 bis 4, **dadurch gekennzeichnet, dass** die Reaktivdestillation in dem Dünnschichtverdampfer bei mindestens einer der folgenden Verfahrensbedingungen durchgeführt wird:
- Mantelbetriebstemperatur von 80 bis 180 °C;
- Kondensatortemperatur von -10° C bis 30 °C;
- Produkttemperatur in der Vorlage von 30 bis 70 °C;
- Produkttemperatur im Auslauf von 75 bis 120 °C; und
- Druck von 50 bis 900 mbar.

6. Verfahren nach einem der Ansprüche 1 bis 5, **dadurch gekennzeichnet, dass** die Reaktionsmischung während der Reaktivdestillation einem Strippvorgang unterworfen wird.

7. Verfahren nach Anspruch 6, **dadurch gekennzeichnet, dass** der Strippvorgang im Gegenstrom zu dem Strom der Reaktionsmischung erfolgt.

8. Verfahren nach Anspruch 7, **dadurch gekennzeichnet, dass** sich an den Verfahrensschritt (g) eine Konfektionierung des erhaltenen Lactamats auf einer Schuppenwalze oder einem Pastilierband anschließt.

9. Verfahren nach einem der Ansprüche 1 bis 8, **dadurch gekennzeichnet, dass** das Verfahren unter inerten Bedingungen durchgeführt wird.

## Claims

1. Process for preparing lactamates by reacting at least one alkoxide with at least one lactam, **characterized in that** a reaction mixture comprising at least one alkoxide and at least one lactam is subjected to a reactive distillation on a thin-film evaporator, wherein the following process steps are run:
(a) providing at least one alcoholic alkoxide solution;
(b) providing at least one lactam;
(c) introducing the at least one lactam into the alcoholic alkoxide solution to obtain a reactant mixture;
(d) homogenizing the reaction mixture obtained in process step (c) at a temperature of 30-70°C which is selected such that there is essentially no reaction of the lactam with the alkoxide, to obtain a homogenized reactant mixture;
(e) feeding the homogenized reactant mixture into a thin-film evaporator, preferably via the top of the thin-film evaporator;
(f) reactively distilling the reactant mixture fed in, optionally with simultaneous stripping of the reactant mixture in countercurrent; and
(g) obtaining the lactamate at the bottom of the thin-film evaporator.

2. Process according to Claim 1, **characterized in that** the lactam corresponds to the general formula 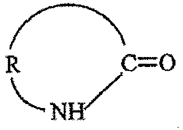 where R is an alkylene group having 3 to 13 carbon atoms.

3. Process according to Claim 1 or 2, **characterized in that** the alkoxide used is an alkali metal alkoxide, alkaline earth metal alkoxide or tetraalkylammonium alkoxide.

4. Process according to any of Claims 1 to 3, **characterized in that** the weight ratio of lactam to alkoxide in the reaction mixture is 1:1 to 40:1.

5. Process according to any of Claims 1 to 4, **characterized in that** the reactive distillation in the thin-film evaporator is performed under at least one of the following process conditions:
- jacket operating temperature from 80 to 180°C;
- condenser temperature from -10°C to 30°C;
- product temperature in the receiver of 30 to 70°C;
- product temperature in the outlet of 75 to 120°C; and
- pressure of 50 to 900 mbar.

6. Process according to any of Claims 1 to 5, **characterized in that** the reaction mixture is subjected to a stripping operation during the reactive distillation.

7. Process according to Claim 6, **characterized in that** the stripping operation is effected in countercurrent to the flow of the reaction mixture.

8. Process according to Claim 7, **characterized in that** process step (g) is followed by finishing of the lactamate obtained on a drum flaker or a pelletizing belt.

9. Process according to any of Claims 1 to 8, which is performed under inert conditions.

## Revendications

1. Procédé pour la préparation de lactamates par mise en réaction d'au moins un alcoolate avec au moins un lactame, **caractérisé en ce que** qu'on soumet un mélange réactionnel, contenant au moins un alcoolate et au moins un lactame, à une distillation réactive sur un évaporateur à couche mince, dans lequel sont effectuées les étapes suivantes de processus :
(a) disposition d'au moins une solution alcoolique d'alcoolate ;
(b) disposition d'au moins un lactame ;
(c) introduction dudit au moins un lactame dans la solution alcoolique d'alcoolate, avec obtention d'un mélange de produits de départ ;
(d) homogénéisation du mélange réactionnel obtenu dans l'étape (c) du processus, à une température de 30 à 70 °C, qui est choisie de manière qu'il ne se produise pratiquement aucune réaction du lactame avec l'alcoolate, avec obtention d'un mélange de produits de départ homogénéisé ;
(e) introduction du mélange de produits de départ homogénéisé dans un évaporateur à couche mince, de préférence par la tête de l'évaporateur à couche mince ;
(f) distillation réactive du mélange de produits de départ introduit, éventuellement avec stripage simultané du mélange de produits de départ à contre-courant ; et
(g) obtention du lactamate au fond de l'évaporateur à couche mince.

2. Procédé selon la revendication 1, **caractérisé en ce que** le lactame correspond à la formule générale 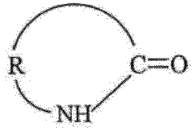 dans laquelle R représente un groupe alkylène ayant de 3 à 13 atomes de carbone.

3. Procédé selon la revendication 1 ou 2, **caractérisé en ce qu'**on utilise comme alcoolate un alcoolate de métal alcalin, de métal alcalino-terreux ou de tétraalkylammonium.

4. Procédé selon l'une quelconque des revendications 1 à 3, **caractérisé en ce que** le rapport pondéral de lactame à alcoolate dans le mélange réactionnel vaut de 1 : 1 à 40 : 1.

5. Procédé selon l'une quelconque des revendications 1 à 4, **caractérisé en ce que** la distillation réactive dans l'évaporateur à couche mince est effectuée dans au moins l'une des conditions de processus suivantes :
- température de fonctionnement de l'enveloppe de 80 à 180 °C ;
- température du condenseur de -10 °C à 30 °C ;
- température du produit dans le récipient collecteur de 30 à 70 °C ;
- température du produit dans le conduit d'évacuation de 75 à 120 °C ; et
- pression de 50 à 900 mbars.

6. Procédé selon l'une quelconque des revendications 1 à 5, **caractérisé en ce que** pendant la distillation réactive le mélange réactionnel est soumis à un processus de stripage.

7. Procédé selon la revendication 6, **caractérisé en ce que** le processus de stripage s'effectue à contre-courant par rapport au courant du mélange réactionnel.

8. Procédé selon la revendication 7, **caractérisé en ce qu'**à l'étape (g) du processus fait suite un façonnage sur un cylindre à lamelles ou une bande pastilleuse du lactamate obtenu.

9. Procédé selon l'une quelconque des revendications 1 à 8, **caractérisé en ce que** le procédé est mis en oeuvre dans des conditions inertes.
